# EUROPÄISCHE PATENTANMELDUNG

(11) **EP 1 958 585 A1**
(43) Veröffentlichungstag der Anmeldung: **20.08.2008**
(21) Anmeldenummer: 07002992.1
(22) Anmeldetag: 13.02.2007
(51) Int. Cl.: A61B 19/00

(54) **Formveränderliche Markereinrichtung**

(71) Anmelder: BrainLAB AG, 85622 Feldkirchen (DE)
(72) Erfinder: Hook, Ingmar, 85622 Feldkirchen (DE); Fleig, Oliver, 85598 Baldham (DE)
(74) Vertreter: Schwabe - Sandmair - Marx

(57) **Zusammenfassung**

Die vorliegende Anmeldung betrifft eine formveränderliche Markereinrichtung zur Anpassung an den menschlichen oder tierischen Körper, mit Markerelementen, wobei die Markereinrichtung ein Verbindungsmittel aufweist, das zumindest einen Teil der Markerelemente so verbindet, dass sie bei Beabstandung voneinander relativ zu einander beweglich sind, um durch die Relativbewegung der Markerelemente die Form der Markereinrichtung dem Verlauf einer gekrümmten Oberfläche möglichst anpassen zu können.

## Beschreibung

Die vorliegende Erfindung betrifft eine formveränderliche Markereinrichtung, die insbesondere an die Oberfläche eines menschlichen oder tierischen Körpers anpassbar ist und/oder daran angelegt werden kann.

Herkömmliche Markereinrichtungen sind nicht formveränderlich, sondern die einzelnen Markerelemente der Markereinrichtung haben eine feste relative Lage zueinander, wie es beispielsweise von so genannten Referenzsternen bekannt ist.

Aufgabe der Erfindung ist es eine Markereinrichtung zu schaffen, die auf einfache und schmerzfreie Weise an einem Patienten anbringbar ist und mit der insbesondere Korrespondenzpunkte nach den Prinzipien der Epipolargeometrie bestimmbar sind.

Vorstehende Aufgabe ist durch den Gegenstand des Anspruches 1 gelöst. Vorteilhafte Weiterbildungen gehen aus den Unteransprüchen hervor.

Die formveränderliche Markereinrichtung umfasst vorzugsweise eine Vielzahl von Markerelementen, die miteinander verbunden sind und relativ zueinander beweglich sind. Die Marker können so gestaltet sein, dass sie für in der medizinischen Analytik verwendete Wellen und/oder Strahlung nicht durchlässig sind (zum Beispiel Röntgenstrahlung), die Wellen und/oder die Strahlung reflektieren (zum Beispiel Infrarotlicht oder Ultraschall) oder selbst Wellen und/oder Strahlung aussenden (zum Beispiel Licht oder Infrarotlicht). Die Wechselwirkung der Markerelemente mit den Wellen und/oder der Strahlung ist durch Detektionseinrichtungen (zum Beispiel Röntgenstrahlungsdetektoren oder lichtempfindliche Sensoren) nachweisbar.

Die Formveränderlichbarkeit der Markereinrichtung wird vorzugsweise durch ein Verbindungsmittel erzielt, das die Markerelemente verbindet. Das Verbindungsmittel kann beispielsweise flexibel sein (zum Beispiel ein Stoff) und/oder kann gelenkig sein (zum Beispiel mechanische Gelenkverbindung zwischen den Markerelementen). Der Abstand zumindest zwischen einem Teil der Markerelemente kann veränderlich sein. Dies ist beispielsweise gegeben, falls das Verbindungsmittel ein flexibles Gewebe ist. Das Verbindungsmittel kann elastisch sein, muss aber nicht. Der Begriff "beweglich" bedeutet hierin, dass die Markerkugeln von einem Menschen ohne Werkzeug mit geringem oder normalem Kraftaufwand relativ zueinander beweglich sind, ohne das Verbindungsmittel dabei zu zerstören oder zu beschädigen.

Vorzugsweise wird die Markereinrichtung eingesetzt, um die Bestimmung eines dreidimensionalen Modells einer anatomischen Struktur aus zwei zweidimensionalen Abbildungen zu erleichtern. Insbesondere soll die Bestimmung von Korrespondenzpunkten erleichtert werden. Korrespondenzpunkte werden bestimmt, um nach den Prinzipien der Epipolargeometrie aus zwei zweidimensionalen Abbildungen einer Struktur aus zwei unterschiedlichen Richtungen die dreidimensionale Lage von Objektpunkten der Struktur bestimmen zu können. Insbesondere soll durch die erfindungsgemäße Markereinrichtung die Bestimmung einer so genannten fundamentalen oder essentiellen Matrix (oder auch Lokalisierungsmatrix) ermöglicht werden, die Eigenschaften der Geometrie beschreiben, die den mindestens zwei Abbildungen zugrunde liegen. Weiterführende Hinweise zur Epipolargeometrie und zur Korrespondenzpunktbestimmung findet man in der parallel eingereichten Patentanmeldung des Anmelders mit dem Titel "Bestimmung des dreidimensionalen Verlaufs des Randes einer anatomischen Struktur", die hiermit in die Offenbarung mit aufgenommen wird und in der Anlage beigefügt ist. Bei herkömmlichen Lokalisierungstechniken zum Bestimmen einer Lokalisierungsmatrix werden starre Objekte verwendet, bei denen die geometrische Beziehung zwischen den Markern von vornherein exakt bekannt ist. Erfindungsgemäß wird eine formveränderliche, insbesondere flexible Markereinrichtung als Lokalisierer verwendet, wobei gemäß einer Ausführungsform die Markerelemente in Röntgenbildern sichtbar sind. Für ein Lokalisierungsverfahren, um insbesondere die Lokalisierungsmatrix nach den Prinzipien der Epipolargeometrie zu bestimmen, ist es von Bedeutung, dass die relative Lage zwischen den Markerelementen in den genutzten Abbildungen gleich bleibt. Die Kenntnis der exakten geometrischen Beziehung zwischen allen Markerelementen ist nicht obligatorisch.

Ein eingesetzter Lokalisierungsalgorithmus, um insbesondere die Lokalisierungsmatrix zu bestimmen, extrahiert beispielsweise die relative Kamerabewegung aus Korrespondenzpunktpaaren, wie dies aus dem Gebiet der "Stereovision" bekannt ist. Derartige Algorithmen sind bekannt und werden verwendet, um eine dreidimensionale Information aus Videoszenen (also eine Abfolge von Bildern, die von einer sich bewegenden Kamera aufgenommen wurden), Satellitenbildern oder Bildern, die von einem speziellen Stereoaufbau erzielt wurden (z.B: zwei Kameras, die parallel ausgerichtet sind und die Bilder simultan erfassen), extrahiert. Beispiele für derartige Algorithmen sind z.B. der Acht-Punkt-Algorithmus (Longuit/Hyggins) oder der Fünf-Punkt-Algorithmus (Stewenius/Engels/Nister), der bevorzugt verwendet wird. Wenn die Algorithmen für Videoaufnahmen oder andere "reale" Bilder, die durch herkömmliche Optik erfasst wurden, verwendet werden, werden üblicherweise Bildmerkmale, wie Kanten und Grau-Farb-Werte verwendet, um automatisch die passenden Korrespondenzen zu finden (z.B. eine Kante eines Verkehrsschildes in einem Bild 1 wird derselben Kante in einem Bild 2 entsprechen). Bei Röntgenbildern wird ein anderer Lösungsansatz bevorzugt, denn üblicherweise ist eine Kanteninformation schwer zu bestimmen oder nicht zuverlässlich, weil zum einen die Bilder eine durchscheinende oder transparente Eigenschaft haben und zum anderen die organischen Objekte abgerundet sind. Letzteres steht im Gegensatz zu den üblichen Videoaufnahmen, die beispielsweise Gebäude oder Autos zeigen, die erkennbare Kanten haben. Erfindungsgemäß werden Markerelemente in das Bild eingefügt, um dadurch auf künstliche Art und Weise "markante" Bildteile zu erzeugen, die zur Verwendung von Korrespondenzpunkten genutzt werden können. Insbesondere wird dadurch eine automatische Detektion der Korrespondenzpunkte ermöglicht.

Vorzugsweise ist bei sinngemäßem Gebrauch der Markereinrichtung (d.h. die Markereinrichtung ist an einem menschlichen oder tierischen Körper angebracht bzw. angepasst) zumindest ein Teil (vorzugsweise ein Großteil) der Markerelemente voneinander beabstandet, während die Markerelemente vorzugsweise auch relativ zueinander beweglich sind. Insbesondere ist das Verbindungsmittel so gestaltet, dass die Markerelemente, wenn die Markereinrichtung ausgebreitet ist, vorgegebene Positionen einnehmen. Die Markereinrichtung ist insbesondere flächig ausgebildet.

Vorzugsweise werden durch das Verbindungsmittel mindestens zwei der Markerelemente auf einem festen Abstand gehalten. Dazu kann das Verbindungsmittel zwischen diesen beiden Markerelementen versteift sein oder ein starres Verbindungsglied aufweisen, an dessen Enden jeweils ein Markerelement angebracht ist. Der bekannte, feste Abstand wird vorzugsweise genutzt, um die Geometrie des abgebildeten Objekts zu kalibrieren, insbesondere zu skalieren. Insbesondere kann so die Lokalisierungsmatrix skaliert werden. Der Abstand zwischen den übrigen Markerelementen kann veränderlich sein.

Vorzugsweise haben die Marker eine sich unterscheidende Form und/oder Größe. Insbesondere gibt es mindestens zwei Gruppen von Markern, wobei die Form und Größe innerhalb der Gruppe gleich ist und sich die Form und/oder Größe der Marker unterscheiden, die zu verschiedenen Gruppen gehören.

Vorzugsweise unterscheiden sich die mindestens zwei Marker, die fest voneinander beabstandet sind, in ihrer Form und/oder Größe von den übrigen Markern oder gehören zu einer Gruppe von Markern, die sich in ihrer Form und/oder Größe von der Mehrzahl der Markerelemente unterscheidet.

Vorzugsweise wird die formveränderliche Markereinrichtung zumindest teilweise um einen Teil eines menschlichen oder tierischen Körpers herum gewunden oder um diesen herum angebracht. Vorzugsweise ergibt sich dann bei einer Aufnahme dieses Körperteils, dass sich ein erster Teil der Markerelemente aus Blickrichtung des Abbildungsgeräts vor dem Teil des menschlichen bzw. tierischen Körpers befindet und ein zweiter Teil dahinter. Vorzugsweise sind die Markerelemente in ihrer Form und/oder Größe und/oder Anordnung charakteristisch unterschiedlich, so dass sich aus der Abbildung ersehen lässt, welche Markerelemente vor dem Teil des Körpers und welche hinter dem Teil des Körpers liegen. Der umgebene Körperteil wird auch als "Innenbereich" bezeichnet, da er innerhalb des von der Markereinrichtung umgebenen Bereichs liegt. Eine charakteristische Anordnung wäre beispielsweise eine Anordnung in Linien, wobei die obere und untere Linie im Vordergrund liegt und die mittlere Linie im Hintergrund. Alternativ oder zusätzlich könnten beispielsweise die im Vordergrund liegenden Markerelemente in einer Zick-Zack-Form angeordnet sein, während die im Hintergrund gelegenen Markerelemente geradlinig angeordnet sind. Eine weitere Alternative wäre, dass die im Vordergrund angeordneten Markerelemente würfelförmig sind, während die im Hintergrund angeordneten Markerelemente kugelförmig sind, so dass sich in der Abbildung quadratische oder runde Flächen ergeben, die eine Identifizierung der Marker als zum Vordergrund oder zum Hintergrund erlauben. Schließlich können beispielsweise die im Hintergrund angeordneten Markerelemente eine deutlich andere Größe haben als die im Vordergrund angeordneten Markerelemente. Auch so lassen sie sich unterscheiden.

Das Verbindungsmittel kann auch so ausgebildet sein, dass es eine Relativbewegung der Markerelemente in eine Richtung leichtgängiger erlaubt als in einer dazu senkrechten Richtung. Beispielsweise kann die Markereinrichtung so ausgebildet sein, dass sich eine Verformung der Markereinrichtung in die Form eines Zylindermantels leicht bewerkstelligen lässt, während eine Relativbewegung der Markerelemente in Richtung der Zylinderachse einen höheren Kraftaufwand erfordert. Auf diese Art und Weise kann der Markereinrichtung eine erhöhte Stabilität gegeben werden.

Bei der folgenden detaillierten Beschreibung von Ausführungsformen der Erfindung werden weitere bevorzugte Merkmale offenbart, die untereinander kombiniert werden können.
- Figur 1: zeigt die Anordnung der Markerelemente bei einer erfindungsgemäßen Markereinrichtung;
- Figur 2: zeigt eine Röntgenaufnahme eines Beckens, um das eine erfindungsgemäße Markereinrichtung gewunden ist;
- Figur 3: zeigt eine weitere Röntgenaufnahme unter denselben Bedingungen wie Figur 2, aber aus einer anderen Richtung.

Gemäß einer Ausführungsform der Erfindung sind die Markerelemente flächig angeordnet und werden über ein flächiges Gewebe verbunden. Insbesondere sind die Markerelemente an vorgegebenen Positionen des flächigen Gewebes angebracht.

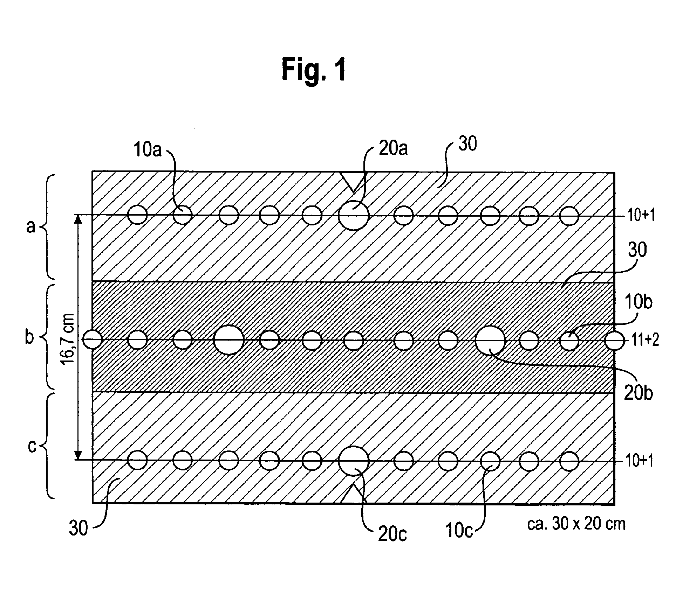

Figur 1 zeigt ein Anordnungsschema der Markerelemente, die als entlang von Streifen a, b und c angebrachte schwarze Kreisflächen 10 und 20 dargestellt sind. Die Markerelemente sind vorzugsweise als Markerkugeln ausgebildet, die in zwei Gruppen unterschiedlichen Durchmessers eingeteilt sind. Die Durchmesser betragen im angegebenen Beispiel 5 mm und 9 mm. Die Markerkugeln entlang eines Streifens sind jeweils um 2,5 cm beabstandet. Die Größen (Durchmesser) der Markerelemente können beliebig gewählt werden. Sie sind insbesondere größer als 1 mm und kleiner als 3 cm. Der Abstand der Markerelemente innerhalb eines Streifens ist insbesondere größer als 2 mm und insbesondere kleiner als 10 cm. Der Abstand zwischen den Mittelpunkten der Markerelemente im oberen Streifen a und unterem Streifen c beträgt im angegebenen Beispiel 16,7 cm. Dies ist rein beispielhaft. Der Abstand ist insbesondere größer als 3 cm und insbesondere kleiner als 30 cm. Die Außenabmessung der in Figur 1 gezeigten Anordnung beträgt ca. 20 x 30 cm und ist ebenfalls rein beispielhaft.

Bei einer Ausführungsform kann das Gewebe mit den Markerelementen in der Art eines Nierengurtes oder in der Art einer Manschette um einen Körperteil eines Patienten (eines Menschen oder Tieres) gewunden werden. Beispielsweise kann das in Figur 1 gezeigte flächige Gewebe 30 in die Gestalt eines Zylindermantels gebracht werden. Dabei sollen die Streifen a und c an der vorderen Hälfte des Zylindermantels liegen, während der Streifen b an der rückwärtigen Hälfte des Zylindermantels liegen soll. Das in Figur 1 gezeigte Gewebe kann also beispielsweise doppellagig sein, wobei die Streifen a und c zu der vorderen Lage gehören und der Streifen b zu der rückwärtigen Lage. Anders ausgedrückt stellen die Streifen a und c eine Ansicht der manschettenförmigen Markereinrichtung von vorne dar und der Streifen b eine Ansicht von hinten dar.

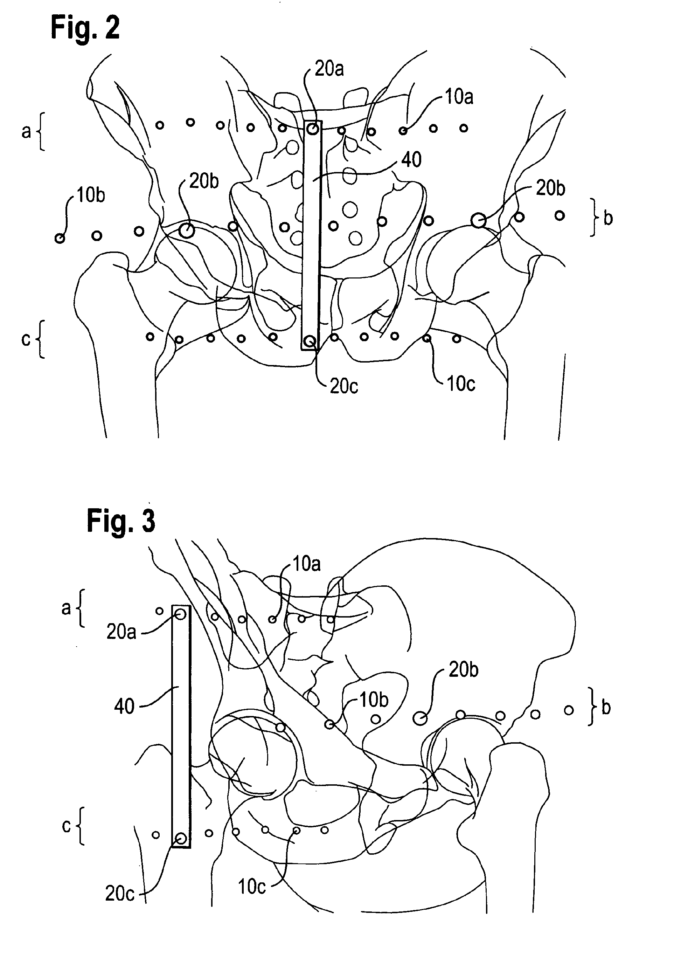

Figur 2 ist eine Röntgenaufnahme eines Beckens. Vor der Röntgenaufnahme wurde ein gemäß Figur 1 gestalteter Gurt um das Becken des Patienten herum gewunden und beispielsweise durch einen Gürtel, durch Knöpfe und/oder aufgrund der elastischen Ausbildung des Gewebes am menschlichen Körper angebracht und/oder durch Spannung gehalten. In Figur 2 sind wiederum als schwarze Kreise die in den Röntgenaufnahmen sichtbaren Markerelemente 10 und 20 in ihrer aus Figur 1 bekannten, charakteristischen Anordnung zu sehen. Sie sind entlang der Linien a, b und c angeordnet, die den Streifen a, b und c der Figur 1 entsprechen. Die Linien sind gut zu erkennen, da vorzugsweise der Abstand der Markerelemente innerhalb einer Linie kleiner ist als der Abstand zwischen den Linien. Auffällig sind die größeren Markerkugeln 20, die sich klar von den kleineren Markerkugeln 10 abheben. Weiter ist ein Stab 40 zu sehen, der in Figur 1 nicht gezeigt ist. Der Stab 40 besteht vorzugsweise aus einem für Röntgenstrahlen zumindest teilweise durchlässigen Werkstoff, z.B. einem Kunststoff, z.B. PVC. Der Stab 40 ist vorzugsweise starr ausgebildet und legt einen festen Abstand zwischen zwei Markerkugeln fest, die sich auf derselben Seite des Körperteils befinden. Im gegebenen Beispiel handelt es sich um die Verbindung der beiden großen Markerkugeln 20a und 20c, die sich auf der Vorderseite des Beckens befinden. Die Verwendung eines Abstandhalters 40 erlaubt eine größenmäßige Kalibrierung bzw. Skalierung der Lokalisierungsmatrix.

Während Figur 2 eine Frontalaufnahme des Beckens von vorne darstellt, ist Figur 3 eine Röntgenaufnahme, die aus Sicht des Betrachters von schräg rechts vorne erfolgt ist. Anders ausgedrückt wurde bei einem ortsfesten Aufnahmegerät das rechte Hüftgelenk nach vorne gedreht.

Die einander in den Figuren 2 und 3 korrespondierenden Markerkugeln sind leicht bestimmbar. Als Ausgangspunkte können die großen Markerkugeln 20 dienen. Die große Markerkugel 20a hat beispielsweise rechts neben sich 5 kleine Markerkugeln 10a. Jeweils die gleichen Markerkugeln wurden in der Figur 2 und 3 mit Bezugszeichen versehen. In Figur 3 ist in der mittleren Reihe b die linke der beiden großen Markerkugeln 20b von Figur 2 zu sehen. Die großen Markerkugeln 20a und 20c sind in beiden Abbildungen dargestellt. Eine zusätzliche Erkennungshilfe bietet der durchscheinend sichtbare Abstandshalter 40. Aufgrund der relativen Lageverschiebung zwischen den Markerkugeln, wenn man Figur 2 und Figur 3 vergleicht, lässt sich die unterschiedliche gegebene Aufnahmegeometrie bestimmen. Wie man erkennen kann, verschiebt sich die mittlere Linie von Figur 2 nach Figur 3 nach rechts relativ zu der oberen und unteren Linie. Dies liegt daran, dass die Markerkugeln des Streifens b hinter dem abgebildeten Becken liegen, während die Markerkugeln des Streifens a und c vor diesem liegen. Durch die Änderung der Abbildungsrichtung verschiebt sich in den Abbildungen scheinbar die Lage der Markerkugeln. In Realita sind jedoch die Markerkugeln ortsfest relativ zu der anatomischen Struktur während der Durchführung der beiden Röntgenaufnahmen, da die Markereinrichtung am Patienten festgeschnallt ist.

Aus der Relativverschiebung beispielsweise der in Figur 3 links abgebildeten Kugeln 20b relativ zu den Kugeln 20a und 20c von Figur 2 nach Figur 3 und aufgrund des bekannten Abstandes zwischen den Markerkugeln 20a und 20c lässt sich die geänderte Abbildungsrichtung bestimmten. Unterstützend können auch noch die Abstände zwischen den Markerkugeln innerhalb einer Linie herangezogen werden, insbesondere wenn es sich bei dem Gewebe um ein flexibles aber nicht elastisches Gewebe handelt.

Zusammengefasst lässt sich aus den Abbildungen der Markerelement Information über die Änderung der Abbildungsbedingungen von Abbildung zu Abbildung, insbesondere über die Änderung der Abbildungsrichtung bestimmen. Insbesondere lassen sich die so genannte essentielle Matrix oder Lokalisierungsmatrix bestimmen, die wesentliche Informationen über die sich von Abbildung zu Abbildung ändernde Abbildungsgeometrie enthält. Ist diese Matrix bestimmt, so können beispielsweise basierend auf dem Prinzip der Epipolargeometrie dreidimensionale Modelle der abgebildeten anatomischen Struktur aus den beiden Abbildungen erstellt werden.

Wie in Figur 2 gezeigt ist, enthält die mittlere Reihe zwei große Markerkugeln. Dies ist nur eine beispielhafte Ausführungsform. Gemäß einer anderen Ausführungsform genügt auch eine größere Markerkugel. Der Vorteil einer Anordnung der größeren Markerkugeln 20b links und rechts der beim Stab 40 liegenden Mitte liegt in der einfacheren Handhabbarkeit. Insbesondere muss der Gurt nicht abhängig davon, ob die linke oder rechte Seite der anatomischen Struktur genauer untersucht werden soll, um eine normal zur und durch die Mitte der Fig. 1 laufende Achse gedreht werden.

Ist ein Aufnahmeprotokoll für die Röntgenaufnahme festgelegt, bei der der zu behandelnde Teil nach vorne oder nach hinten zu drehen ist, beispielsweise nach vorne, so lässt sich automatisch aus der Verschiebung der Markerkugelreihen gegeneinander bestimmen, welche Seite die zu behandelnde Seite ist. Anders ausgedrückt durch das Vorzeichen des aus den Abbildungen bestimmten Drehwinkels zwischen den beiden Abbildungen lässt auf die zu behandelnde Seite aufgrund des Aufnahmeprotokolls schließen. Dies kann im Rahmen einer Auswertungssoftware ausgenutzt werden.

## Patentansprüche

1. Formveränderliche Markereinrichtung zur Anpassung an den menschlichen oder tierischen Körper, mit Markerelementen (10, 20),
wobei die Markereinrichtung ein Verbindungsmittel (30) aufweist, das zumindest einen Teil der Markerelemente (10, 20) so verbindet, dass sie bei Beabstandung voneinander relativ zu einander beweglich sind, um durch die Relativbewegung der Markerelemente die Form der Markereinrichtung dem Verlauf einer gekrümmten Oberfläche möglichst anpassen zu können.

2. Formveränderliche Markereinrichtung nach Anspruch 1, bei welchem das Verbindungsmittel (30) zwischen mindestens zwei der Markerelemente (20a, 20c') einen festen Abstand hält.

3. Formveränderliche Markereinrichtung nach Anspruch 2, wobei die mindestens zwei fest beabstandeten Markerelemente (20a, 20c) eine Form und/oder Größe aufweisen, die sich von anderen Markerelementen (10) unterscheidet.

4. Formveränderliche Markereinrichtung nach einem der vorhergehenden Ansprüche, wobei der Abstand zwischen mindestens zwei Markerelementen veränderlich ist.

5. Formveränderliche Markereinrichtung nach einem der vorhergehenden Ansprüche, wobei zumindest ein Teil der Markerelemente (20) eine in den Abbildungen nachweisbare Form und/oder Größe aufweisen, die sich von den übrigen Markerelementen (10) unterscheidet.

6. Formveränderliche Markereinrichtung nach einem der vorhergehenden Ansprüche, bei welcher die Anordnung der Markerelemente dergestalt ist, dass, falls die Markereinrichtung eine gekrümmte, in sich geschlossene Fläche bildet, bei Draufsicht auf die gekrümmte Fläche in Normalenrichtung zumindest ein Teil der Markerelemente (10, 20) nicht hintereinander liegen

7. Formveränderliche Markereinrichtung nach Anspruch 6, bei welcher die gekrümmte Fläche einen Innenbereich umgibt, bezüglich dem aus einer Blickrichtung ein Teil der Markerelemente (10a, 20a, 10c, 20c) vor dem Innenbereich und ein anderer Teil der Markerelemente (20b, 10b) hinter dem Innenbereich liegt, wobei die Anordnung und/oder Größe und/oder Form der im Vordergrund angeordneten Markerelemente sich in charakteristischer Weise von denen im Hintergrund angeordneten Markerelementen (10b, 20b) unterscheiden.

8. Formveränderliche Markereinrichtung nach Anspruch 7, bei welcher die Anordnung der Markerelemente sich charakteristisch so unterscheidet, dass sie bei Draufsicht aus der Blickrichtung entlang voneinander beabstandeten Linien angeordnet sind

9. Formveränderliche Markereinrichtung nach einem der vorhergehenden Ansprüche, bei welcher das Verbindungsmittel aus einem flexiblen Material ist und/oder eine mechanische Gelenkverbindung aufweist, die gelenkig die Markerelemente durch Verbindungselemente miteinander verbindet.

10. Formveränderliche Markereinrichtung nach Anspruch 9, bei welcher die Länge des flexiblen Materials oder der Verbindungselemente zwischen den Markerelementen (10, 20) zumindest in etwa konstant ist.

11. Formveränderliche Markereinrichtung nach Anspruch 10, bei welcher das flexible Material oder die mechanische Gelenkverbindung so ausgestaltet ist, dass die Markerelemente in einer Ebene leichter beweglich sind als in einer zu der Ebene senkrechten Richtung.
